# EUROPEAN PATENT APPLICATION

(11) **EP 1 621 195 A2**
(43) Date of publication of application: **01.02.2006**
(21) Application number: 05017203.0
(22) Date of filing: 03.07.2002
(51) Int. Cl.: A61K 31/435, A61P 25/28

(54) **The use of pyridinic NK-1 receptor antagonists for the treatment of brain, spinal or nerve injury**

(30) Priority: 10.07.2001 EP 01116812
(62) Divisional of application: 02764617.3
(71) Applicant: F.HOFFMANN-LA ROCHE AG, 4070 Basel (CH)
(72) Inventor: Hoffmann, Torsten, 79576 Weil am Rhein (DE); Sleight, Andrew, 68400 Riedisheim (FR); Vankan, Pierre, 4125 Riehen (CH); Vink, Robert, Pasadena, South Australia 5042 (AU); Nimmo, Alan John, Townsville, Queensland 4810 (AU)
(74) Representative: Poppe, Regina

(57) **Abstract**

The invention relates to the use of an NK-1 receptor antagonist, optionally in combination with a magnesium salt, for the treatment and/or prevention of brain, spinal or nerve injury, wherein said NK-1 receptor antagonist is a compound of the general formula 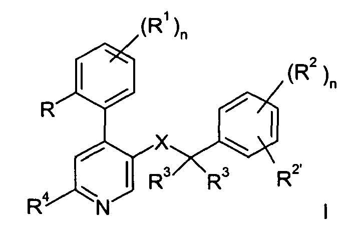
wherein the meanings of R, R¹, R², R^{2'}, R³, R⁴ are explained in the specification and the pharmaceutically acceptable acid addition salts and the prodrugs thereof either alone or in combination with a magnesium salt. Exemplified is the use of N-(3,5-bis-trifluoromethylbenzyl)-N-methyl-6-(4-methyl-piperazin-1-yl)-4-o-tolyl-nicotinamide. The invention also relates to pharmaceutical composition comprising one or more such NK-1 receptor antagonists, optionally in combination with a magnesium salt, and a pharmaceutically acceptable excipient for the treatment and/or prevention of brain, spinal or nerve injury.

## Description

The present invention concerns NK-1 receptor antagonists and their use for the treatment and/or prevention of brain, spinal or nerve injury.

Brain, spinal or nerve injury occurs in connection with accidents and results often in the development of motor and cognitive deficits that contribute to the significant morbidity experienced by survivors of accidents. Due to their life style younger members of the society are particularly prone to such accidents. The financial loss caused by the injuries incurred by such accidents is significant. Therefore, any means to increase the survival and an improved recovery of nerve damages incurred in accidents is of great benefit to society.

Neurokinin-1 (NK-1) or substance P is a naturally occurring undecapeptide belonging to the tachykinin family of peptides, the latter being so-named because of their prompt contractile action on extravascular smooth muscle tissue. The receptor for neurokinin-1 or substance P is a member of the superfamily of G protein-coupled receptors and is named NK-1 receptor. This receptor is widely distributed throughout the mammalian nervous system (especially brain and spinal ganglia) and is also present in the circulatory system and in peripheral tissues (especially the duodenum, the jejunum and the genito-urinary tract). The receptor is believed to be involved in the regulation of a number of diverse biological processes as outlined below.

The central and peripheral actions of the mammalian tachykinin substance P have been associated with numerous inflammatory conditions including migraine, rheumatoid arthritis, asthma, and inflammatory bowel disease as well as mediation of the emetic reflex and the modulation of central nervous system (CNS) disorders such as Parkinson's disease (Neurosci. Res., 7, 187-214, (1996)), anxiety (Can. J. Phys., 75, 612-621, (1997)) and depression (Science, 281, 1640-1645, (1998)).

Evidence for the usefulness of tachykinin receptor antagonists in pain, headache, especially migraine, Alzheimer's disease, multiple sclerosis, attenuation of morphine withdrawal, cardiovascular changes, edema, such as edema caused by thermal injury, chronic inflammatory diseases such as rheumatoid arthritis, asthma/bronchial hyperreactivity and other respiratory diseases including allergic rhinitis, inflammatory diseases of the gut including ulcerative colitis and Crohn's disease, ocular injury and ocular inflammatory diseases reviewed in "Tachykinin Receptor and Tachykinin Receptor Antagonists", J. Auton. Pharmacol., 13, 23-93, (1993).

Furthermore, neurokinin-1 receptor antagonists are being developed for the treatment of a number of physiological disorders associated with an excess or imbalance of tachykinin, in particular substance P. Examples of conditions in which substance P has been implicated include disorders of the central nervous system such as anxiety, depression and psychosis (International Patent Application, Publication Nos. WO 95/16679, WO 95/18124 and WO 95/23798).

The neurokinin-1 receptor antagonists are further believed to be useful for the treatment of motion sickness and for treatment induced vomiting.

The reduction of cisplatin-induced emesis by a selective neurokinin-1-receptor antagonist is described in The New England Journal of Medicine, Vol. 340, No. 3, 190-195, (1999).

Furthermore, US Patent No. 5,972,938 describes a method for treating a psychoimmunologic or a psychosomatic disorder by administration of a tachykinin receptor, such as the NK-1 receptor antagonist.

The usefulness of neurokinin 1 receptor antagonists for the treatment of certain forms of urinary incontinence is furthermore described in Neuropeptides, 32(1), 1-49, (1998) and Eur. J. Pharmacol., 383(3), 297-303, (1999).

European Patent Application EP-A-721 778 relates to the use of a specific group of compounds in the manufacture of a medicament for the treatment of a disorder selected from stroke, epilepsy head trauma, spinal cord trauma, ischemic neuronal damage from stroke or vascular occlusion, excitoxic neuronal damage and amyotrophic sclerosis in a mammal.

NK-1 receptor antagonists have been reported to have also a beneficial effect in the therapy of traumatic brain injury (International Patent Application No. PCT/AU01/00046, Publication No. WO 01/52844). The beneficial effects of the NK-1 receptor antagonist N-acetyl-L-tryptophan for the improvement of the neurological outcome following traumatic brain injury (TBI) has been reported in an oral disclosure by Prof. Nimmo at the International Tachykinin Conference 2000 in La Grande Motte, France, October 17-20, 2000 (Authors: Nimmo A.J., Bennett C.J., Hu X., Cernak I., Vink R.).

The use of NK-1 receptor antagonists for the treatment or prevention of chronic nonbacterial prostatitis and prostatodynia has been described in International Patent Publication No. WO 99/59583.

International Patent Publication No. WO 01/01922 describes the use of substance P antagonists in the treatment of the adenocarcinomas, particularly genito-urinary tract neoplasms such as prostatic carcinoma.

It has now been found that the selective, brain penetrant NK-1 receptor antagonist of the general formula 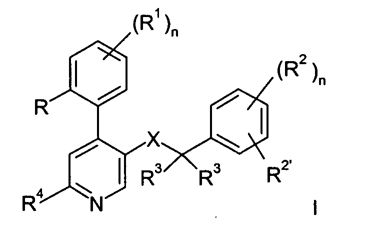 wherein
- R: is hydrogen, lower alkyl, lower alkoxy, halogen or trifluoromethyl;
- R¹: is hydrogen or halogen; or
- R and R¹: may be together -CH=CH-CH=CH-;
- R² and R^{2'}: are independently from each other hydrogen, halogen, trifluoromethyl, lower alkyl, lower alkoxy or cyano; or
- R² and R^{2'}: may be together -CH=CH-CH=CH-, optionally substituted by one or two substituents selected from lower alkyl, halogen or lower alkoxy;
- R³: is, independently from each other if occurring twice, hydrogen, lower alkyl or may, if occurring twice, form together with the carbon atom to which they are attached a cycloalkyl group;
- R⁴: is hydrogen, -N(R⁵)₂, -N(R⁵)(CH₂)ₙOH, -N(R⁵)S(O)₂-lower alkyl, -N(R⁵)S(O)₂₋phenyl, -N=CH-N(R⁵)₂, -N(R⁵)C(O)R⁵, a cyclic tertiary amine of the group 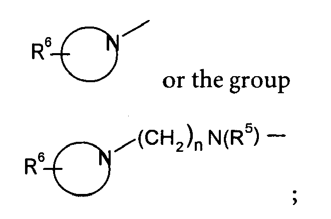
- or R⁴: is -(C≡C)ₘR⁷ or -(CR'=CR")ₘR⁷
wherein R⁷ is
a) halogen,
b) cyano, or the following groups:
c) -(CR'R")ₘ-R⁸,
d) -C(O)NR'R",
e) -C(O)O(CH₂)ₙR⁸,
f) -C(O)R⁸,
g) -N(OH)-(CH₂)ₙR⁸,
h) -NR'C(O)-(CH₂)ₙR⁸,
i) -N[C(O)-R']₂,
j) -OR⁹,
k) -(CH₂)ₙ-SR⁹, -(CH₂)ₙ-S(O)R⁹, or -(CH₂)ₙ-S(O)₂R⁹,
l) aryl, optionally substituted by one or more substituents, selected from halogen, trifluoromethyl, lower alkyl, lower alkoxy, cyano, hydroxy, -NR'R", nitro, -(CH₂)ₘOR', -C(O)NR'R", -C(O)OR' or -C(O)R',
m) is a five or six membered heteroaryl group, containing one to four heteroatoms, selected from N, O or S and may be optionally substituted by one or more substituents, selected from halogen, trifluoromethyl, lower alkyl, lower alkoxy, cyano, hydroxy, -NR'R", nitro, -(CH₂)ₘOR', -C(O)OR', -C(O)NR'R" or -C(O)R', n) is a five or six membered saturated cyclic tertiary amine of the group 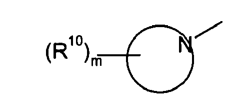 which may contain one additional heteroatom, selected from N, O or S,
R'/R" are independently from each other hydrogen, hydroxy, lower alkyl, cycloalkyl or aryl, wherein the lower alkyl, cycloalkyl or aryl group may be optionally substituted by one or more substituents, selected from halogen, trifluoromethyl, lower alkyl, lower alkoxy, cyano, hydroxy, -NR"'R"", nitro, -(CH₂)ₘOR"', -C(O)NR"'R"", -C(O)OR"' or -C(O)R"',
R"'/R"" are independently from each other hydrogen, lower alkyl, cycloalkyl or aryl,
R⁸ is hydrogen, cyano, hydroxy, halogen, trifluoromethyl, -C(O)OR', -OC(O)R' or aryl, optionally substituted by one or more substituents, selected from halogen, trifluoromethyl, lower alkyl, lower alkoxy, cyano, hydroxy, -NR'R", nitro, -(CH₂)ₘOR', -C(O)NR'R", -C(O)OR' or -C(O)R', or is a five or six membered heteroaryl group, containing one to four heteroatoms, selected from N, O or S and may be optionally substituted by one or more substituents, selected from halogen, trifluoromethyl, lower alkyl, lower alkoxy, cyano, hydroxy, -NR'R", nitro, -(CH₂)ₘOR', -C(O)NR'R", -C(O)OR' or -C(O)R',
R⁹ is hydrogen, lower alkyl, trifluoromethyl, or aryl, wherein the lower alkyl or aryl group may be optionally substituted by one or more substituents, selected from halogen, trifluoromethyl, lower alkyl, lower alkoxy, cyano, hydroxy, -NR'R", nitro, -C(O)NR'R", -(CH₂)ₘOR', -C(O)OR' or -C(O)R', or is a five or six membered heteroaryl group, containing one to four heteroatoms, selected from N, O or S and may be optionally substituted by one or more substituents, selected from halogen, trifluoromethyl, lower alkyl, lower alkoxy, cyano, hydroxy, -NR'R", nitro, -(CH₂)ₘOR', -C(O)NR'R", -C(O)OR' or -C(O)R',
R¹⁰ is -C(O)-(CH₂)ₘOH or an oxo group;
- or R⁴: is an N-oxide of the general formula 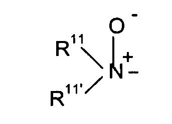
wherein R¹¹ and R^{11'} are independently from each other -(CH₂)ₚOR¹² or lower alkyl, wherein R¹² is hydrogen, lower alkyl or phenyl;
or
R¹¹ and R^{11'} form together with the N-atom to which they are attached a cyclic tertiary amine of the group 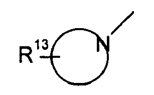
wherein R¹³ is hydrogen, hydroxy, lower alkyl, lower alkoxy, -(CH₂)ₚOH, -COOR³, -CON(R³)₂, -N(R³)CO-lower alkyl or -C(O)R³;
- R⁵: is, independently from each other, hydrogen, C₃-₆-cycloalkyl, benzyl, phenyl or lower alkyl;
- R⁶: is hydrogen, hydroxy, lower alkyl, -(CH₂)ₙCOO-lower alkyl, -N(R⁵)CO-lower alkyl, hydroxy-lower alkyl, cyano, (CH₂)ₙO(CH₂)ₙOH, -CHO or a 5-or 6 membered heterocyclic group, optionally bonded via an alkylene group;
- X: is -C(O)N(R⁵)-, -(CH₂)ₘO-, -O(CH₂)ₘ-, -(CH₂)ₘN(R⁵)-, -N(R⁵)C(O)-, or -N(R⁵)(CH₂)ₘ-;
- n: is 0, 1, 2, 3 or 4;
- m: is 1 or 2; and
- p: is 1, 2, or 3;
and the pharmaceutically acceptable acid addition salts and the prodrugs thereof are particularly suitable for the treatment and/or prevention of brain, spinal or nerve injury.

The present invention relates to the use of an NK-1 receptor antagonist of the general formula (I) for the manufacture of a medicament for the treatment and/or prevention of brain, spinal or nerve injury.

The invention also relates to a method of treatment and/or prevention of brain, spinal or nerve injury in a mammal, including a human, by administering an effective amount of an NK-1 receptor antagonist of the general formula (I) and a pharmaceutically acceptable excipient.

The invention also relates to a pharmaceutical composition comprising one or more NK-1 receptor antagonists and a pharmaceutically acceptable excipient for the treatment and/or prevention of brain, spinal or nerve injury. Said NK-1 receptor antagonist may be present in the form of a pharmaceutically acceptable acid addition salt or may be present in the form of a prodrug, preferably in the form of an N-oxide.

The usefulness of the present invention is supported by the experimental results provided below, which results are presented in the following figures.
Fig. 1 demonstrates the significantly better motor outcome in the Rotarod Motor Score after brain injury in animals administered the NK-1 receptor antagonist compared to animals treated with either saline or MK801.
Fig. 2 shows the significantly better protection against loss of cognitive function (Barnes Cognitive Score) after brain injury in animals treated with the NK-1 receptor antagonist compared to animals treated with either saline or MK801.
Fig. 3 shows the reduced Evans Blue penetration in animals treated with the NK-1 receptor antagonist compared to animals treated with either saline or MK801 after traumatic brain injury.

It has been shown previously (International Patent Application No. PCT/AU01/00046) that the neuroprotective action of NK-1 receptor antagonists can be enhanced with the addition of pharmacologic doses of magnesium to the i.v. solution. Therefore, the NK-1 receptor antagonist as used in accordance with the present invention, such as N-(3,5-bis-trifluoromethyl-benzyl)-N-methyl-6-(4-methyl-piperazin-1-yl)-4-o-tolyl-nicotinamide, is preferably co-administered with pharmacologic doses of magnesium salts (10-100 mg/kg) in order to enhance the neuroprotective properties

The following definitions of the general terms used in the present description apply irrespective of whether the terms in question appear alone or in combination.

The term "treatment" in the phrase "treatment and/or for the prevention of brain, spinal or nerve injury" refers to any application of a NK-1 receptor antagonist within minutes to hours after the impact leading to a brain, spinal or nerve injury in a living subject (e.g. a mammal or a human being). The term "prevention" refers to any prophylactic treatment of a subject made in connection with a possible or an expected impact which may lead to a brain, spinal or nerve injury in said subject. Said prophylactic treatment may be administered immediately before the impact within minutes, within one to 24 hours, or within one to several days before the expected impact. The said administration can occur once or repeatedly (preferably at regular intervals) before the expected impact.

The term "selective" in the phrase "selective, brain penetrant NK-1 receptor antagonist" refers to a 100-fold to 10'000-fold higher affinity of the said antagonist to the said NK-1 receptor compared to its affinity to either the NK-2 receptor and/or the NK-3 receptor. The term "brain penetrant" in said phrase refers to the fact that the NK-1 receptor antagonists used in accordance with the present invention show a good brain penetration, viz. are able to cross the blood-brain barrier (BBB). This is in contrast to N-acetyl-L-tryptophan which shows only a very reduced brain penetration. The preferred compounds used in accordance with the present invention diplay both superior anxiolytic and antidepressive activity and are also able to cross the BBB. The animals treated with the preferred compounds in the treatment and/or for the prevention of brain, spinal or nerve injury show therefore also a markedly reduced posttraumatic depression.

As used herein, the term "lower alkyl" denotes a saturated straight- or branched-chain alkyl group containing from 1 to 7 carbon atoms, for example, methyl, ethyl, propyl, isopropyl, n-butyl, i-butyl, 2-butyl, t-butyl and the like. Preferred lower alkyl groups are groups with 1 to 4 carbon atoms.

The term "lower alkoxy" denotes a group wherein the alkyl residues are as defined above and which is attached via an oxygen atom.

The term "halogen" denotes chlorine, iodine, fluorine and bromine.

The term "cycloalkyl" denotes a saturated carbocyclic group containing 3 to 6 carbon atoms.

The term "cyclic tertiary amine" denotes, for example, pyrrolidin-1-yl, imidazol-1-yl, piperidin-1-yl, piperazin-1-yl, morpholin-4-yl, thiomorpholin-4-yl, 1-oxo-thiomorpholin-4-yl, 1,1-dioxo-thiomorpholin-4-yl, 2,3-dihydro-[1,4]oxazin-4-yl, or [1,2,4]triazol-1-yl.

The term "five or six membered heteroaryl group, containing one to four heteroatoms, selected from N, O or S" denotes, for example, the following groups: pyrrol-1-yl, imidazol-1 or 2-yl, pyrazol-1-yl, pyridin-2, 3 or 4-yl, pyrazinyl, pyrimidinyl, pyridazinyl, isothiazolyl, isoxazolyl, thienyl, 1,2,3-triazolyl, 1,2,4-oxadiazolyl, tetrahydropyridinyl, isoxazolyl or furyl.

The term "five or six membered saturated cyclic tertiary amine" denotes, for example, pyrrolidinyl, imidazolidinyl, pyrazolidinyl, piperidinyl, piperazinyl, morpholinyl, thiomorpholinyl, thiomorpholin-1,1-dioxo or thiomorpholin-1-oxo.

The term "5 or 6 membered heterocyclic group" denotes, for example pyridinyl, pyrimidinyl, oxadiazolyl, triazolyl, tetrazolyl, thiazolyl, thienyl, furyl, pyranyl, pyrrolyl, imidazolyl, pyrazolyl, isothiazolyl, piperazinyl or piperidyl.

The term "aryl" denotes a monocyclic aromatic hydrocarbon radical or a bicyclic or tricyclic ring system in which at least one ring is aromatic, preferred are phenyl, benzyl or naphthyl rings.

The term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methanesulfonic acid, p-toluenesulfonic acid and the like.

The term "a pharmacologic dose of magnesium" means a dosage of a magnesium provided by any suitable means such as by adding a non-toxic magnesium salt such as magnesium chloride, magnesium sulphate, magnesium oxalate, magnesium gluconate, etc., whereby the said magnesium is administered to the patient either seperately or in combination with the NK-1 receptor antagonist. The dosage of the magnesium administered is in the range of 0.1 to 30 mg/kg body weight.

Preferred compounds for the claimed use are the exemplary compounds in which X in general formula (I) is -C(O)N(R⁵)- and wherein R⁵ is methyl, ethyl or cyclopropyl, for example the following compounds:
N-(3,5-bis-trifluoromethyl-benzyl)-N-methyl-4-o-tolyl-nicotinamide,
N-(3,5-bis-trifluoromethyl-benzyl)-N-methyl-4-(2-chloro-phenyl)-nicotinamide,
N-(3,5-bis-trifluoromethyl-benzyl)-N-methyl-4-(2-trifluoromethyl-phenyl)-nicotinamide,
N-(3,5-bis-trifluoromethyl-benzyl)-N-methyl-4-(2-fluoro-phenyl)-nicotinamide,
N-(3,5-bis-trifluoromethyl-benzyl)-N-methyl-4-(2-methoxy-phenyl)-nicotinamide,
N-(3,5-bis-trifluoromethyl-benzyl)-N-methyl-4-phenyl-nicotinamide,
N-(3,5-bis-trifluoromethyl-benzyl)-N-ethyl-4-o-tolyl-nicotinamide,
N-(3,5-bis-trifluoromethyl-benzyl)-N-cyclopropyl-4-o-tolyl-nicotinamide,
N-[1-(3,5-bis-trifluoromethyl-phenyl)-ethyl]-N-methyl-4-o-tolyl-nicotinamide,
N-(3,5-di-fluorobenzyl)-N-methyl-4-o-tolyl-nicotinamide,
N-(3,5-di-chlorobenzyl)-N-methyl-4-o-tolyl-nicotinamide,
N-(3,5-bis-trifluoromethyl-benzyl)-N-methyl-6-(4-methyl-piperazin-l-yl)-4-o-tolyl-nicotinamide,
2'-methyl-5-(4-methyl-piperazin-1-yl)-biphenyl-2-carboxylic acid-(3,5-bis-trifluoromethyl-benzyl)-methyl-amide,
N-(3,5-bis-trifluoromethyl-benzyl)-N-methyl-6-(4-methyl-piperazin-1-yl)-4-naphthalen-1-yl-nicotinamide,
(4-{5-[(3,5-bis-trifluoromethyl-benzyl)-methyl-carbamoyl]-4-o-tolyl-pyridin-2-yl}-piperazin-1-yl)-acetic acid ethyl ester,
5'-[(3,5-bis-trifluoromethyl-benzyl)-methyl-carbamoyl]-4'-o-tolyl-3,4,5,6-tetrahydro-2H-[1,2']bipyridinyl-4-carboxylic acid ethyl ester,
N-(3,5-bis-trifluoromethyl-benzyl)-N-methyl-6-(4-propyl-piperazin-1-yl)-4-o-tolyl-nicotinamide,
(RS)-6-[3-(acetyl-methyl-amino)-pyrrolidin-1-yl]-N-(3,5-bis-trifluoromethylbenzyl)-N-methyl-4-o-tolyl-nicotinamide,
N-(3,5-bis-trifluoromethyl-benzyl)-N-methyl-6-[methyl-(2-morpholin-4-yl-ethyl)-amino]-4-o-tolyl-nicotinamide,
N-(3,5-bis-trifluoromethyl-benzyl)-N-methyl-6-morpholin-4-yl-4-o-tolyl-nicotinamide,
N-(3,5-bis-trifluoromethyl-benzyl)-N-methyl-6-thiomorpholin-4-yl-4-o-tolyl-nicotinamide,
N-(3,5-bis-trifluoromethyl-benzyl)-N-methyl-6-(1-oxo-1λ⁴-thiomorpholin-4-yl)-4-o-tolyl-nicotinamide,
N-(3,5-bis-trifluoromethyl-benzyl)-6-(1,1-dioxo-1λ⁶-thiomorpholin-4-yl)-N-methyl-4-o-tolyl-nicotinamide,
N-(3,5-bis-trifluoromethyl-benzyl)-N-methyl-6-piperazin-1-yl-4-o-tolyl-nicotinamide,
N-(3,5-bis-trifluoromethyl-benzyl)-6-[4-(2-hydroxy-ethyl)-piperazin-1-yl]-N-methyl-4-o-tolyl-nicotinamide,
N-(3,5-bis-trifluoromethyl-benzyl)-6-(4-cyanomethyl-piperazin-1-yl)-N-methyl-4-o-tolyl-nicotinamide,
N-(3,5-bis-trifluoromethyl-benzyl)-6-{4-[2-(2-hydroxy-ethoxy)-ethyl]-piperazin-1-yl}-N-methyl-4-o-tolyl-nicotinamide,
N-(3,5-bis-trifluoromethyl-benzyl)-N-methyl-6-(4-[1,2,4]oxadiazol-3-yl-methyl-piperazin-1-yl)-4-o-tolyl-nicotinamide,
N-(3,5-bis-trifluoromethyl-benzyl)-N-methyl-6-[4-(5-oxo-4,5-dihydro-1H-[1,2,4]triazol-3-yl-methyl)-piperazin-1-yl]-4-o-tolyl-nicotinamide,
N-(3,5-bis-trifluoromethyl-benzyl)-6-(4-formyl-piperazin-1-yl)-N-methyl-4-o-tolyl-nicotinamide, and
N-methyl-N-(2-methyl-naphthalen-1-yl-methyl)-6-morpholin-4-yl-4-o-tolyl-nicotinamide.

Further preferred compounds for the claimed use are the exemplary compounds in which X in general formula (I) is -N(R⁵)-CO- and wherein R⁵ is hydrogen or methyl, for example the following compounds:
2-(3,5-bis-trifluoromethyl-phenyl)-N-methyl-N-[6-(4-methyl-piperazin-1-yl)-4-o-tolyl-pyridin-3-yl]-isobutyramide,
2-(3,5-bis-trifluoromethyl-phenyl)-N-[4-(2-chloro-phenyl)-6-(4-methyl-piperazin-1-yl)-pyridin-3-yl]-N-methyl-isobutyramide,
2-(3,5-bis-trifluoromethyl-phenyl)-N-[4-(4-fluoro-2-methyl-phenyl)-6-(4-methyl-piperazin-1-yl)-pyridin-3-yl]-N-methyl-isobutyramide,
2-(3,5-bis-trifluoromethyl-phenyl)-N-[4-(2-chloro-phenyl)-pyridin-3-yl]-N-methyl-isobutyramide,
2-(3,5-bis-trifluoromethyl-phenyl-N-methyl-N-(4-o-tolyl-pyridin-3-yl)-isobutyramide,
2-(3,5-bis-trifluoromethyl-phenyl)-N-(4-o-tolyl-pyridin-3-yl)-isobutyramide,
2-(3,5-bis-trifluoromethyl-phenyl)-N-methyl-N-(4-o-tolyl-pyridin-3-yl)-acetamide,
2-(3,5-bis-trifluoromethyl-phenyl)-N-methyl-N-(4-o-tolyl-pyridin-3-yl)-propionamide,
2-(3,5-bis-trifluoromethyl-phenyl)-N-methyl-N-(6-morpholin-4-yl-4-o-tolyl-pyridin-3-yl)-isobutyramide,
2-(3,5-bis-trifluoromethyl-phenyl)-N-[4-(2-chloro-phenyl)-6-morpholin-4-yl-pyridin-3-yl] -N-methyl-isobutyramide,
2-(3,5-bis-trifluoromethyl-phenyl)-N-methyl-N-{6-[methyl-(2-morpholin-4-yl-ethyl)-amino] -4-o-tolyl-pyridin-3-yl}-isobutyramide,
2-(3,5-bis-trifluoromethyl-phenyl)-N-methyl-N-[6-(4-pyrimidin-2-yl-piperazin-1-yl)-4-o-tolyl-pyridin-3-yl]-isobutyramide,
2-(3,5-bis-trifluoromethyl-phenyl)-N-(6-morpholin-4-yl-4-o-tolyl-pyridin-3-yl)-isobutyramide,
2-(3,5-bis-trifluoromethyl-phenyl)-N-[4-(2-chloro-phenyl)-6-dimethylamino-pyridin-3-yl] -isobutyramide,
2-(3,5-bis-trifluoromethyl-phenyl)-N-methyl-N-(6-piperazin-1-yl-4-o-tolyl-pyridin-3-yl)-isobutyramide,
2-(3,5-bis-trifluoromethyl-phenyl)-N-(4-hydroxy-4'-o-tolyl-3,4,5,6-tetrahydro-2H-[1,2']bipyridinyl-5'-yl)-N-methyl-isobutyramide,
2-(3,5-bis-trifluoromethyl-phenyl)-N-{6-[(2-hydroxy-ethyl)-methyl-amino]-4-o-tolyl-pyridin-3-yl}-N-methyl-isobutyramide,
(R)-2-(3,5-bis-trifluoromethyl-phenyl)-N-[6-(3-hydroxy-pyrrolidin-1-yl)-4-o-tolyl-pyridin-3-yl]-N-methyl-isobutyramide,
2-(3,5-bis-trifluoromethyl-phenyl)-N-methyl-N-(6-morpholin-4-yl-4-o-tolyl-pyridin-3-yl)-acetamide, and
[2-(3,5-bis-trifluoromethyl-phenyl)-2-methyl-propyl]-[4-(4-fluoro-2-methylphenyl)-6-(4-methyl-piperazin-1-yl)-pyridin-3-yl]-methylamine.

The methods for the preparation of the above-mentioned compounds is described in detail in EP-A-1,035,115. Also provided are values for the affinity of selected compounds to the NK-1 receptor, given as pKi, whereby the pKi value for preferred compounds is in the range of 8.00 to 9.80. EP-A-1,035,115 provides furthermore proposals for suitable formulations of NK-1 receptor antagonists, which are also suitable for the use as claimed in the present patent specification.

Methods for the preparation of additional compounds falling within the scope of general formula (I), which compounds are also suitable for the claimed uses are described in International Patent Application No. PCT/EP01/08432 filed July 7, 2001 based on European Patent Application No. 00115846.8 filed July 24, 2000. Examples of such compounds are:
A) compound of general formula (I), in which X is -C(O)N(R⁵)- and R⁵ is methyl, ethyl or cyclopropyl such as:
   N-(3,5-bis-trifluoromethyl-benzyl)-N-methyl-4-o-tolyl-6-[1,2,4]triazol-1-yl-nicotinamide,
   N-(3,5-bis-trifluoromethyl-benzyl)-6-(2-hydroxy-ethylamino)-N-methyl-4-o-tolyl-nicotinamide,
   4-hydroxy-4'-o-tolyl-3,4,5,6-tetrahydro-2H-[1,2']bipyridinyl-5'-carboxylic acid (3,5-bis-trifluoromethyl-benzyl)-methyl-amide,
   4-(2-hydroxy-ethoxy)-4'-o-tolyl-3,4,5,6-tetrahydro-2H-[1,2']bipyridinyl-5'-carboxylic acid (3,5-bis-trifluoromethyl-benzyl)-methyl-amide,
   (R)-N-(3,5-bis-trifluoromethyl-benzyl)-6-(3-hydroxy-pyrrolidin-1-yl)-N-methyl-4-o-tolyl-nicotinamide,
   4'-(2-chloro-phenyl)-4-hydroxy-3,4,5,6-tetrahydro-2H-[1,2']bipyridinyl-5'-carboxylic acid (3,5-bis-trifluoromethyl-benzyl)-methyl-amide.
B) compound of general formula (I), in which X is -N(R⁵)-C(O)- and R⁵ is hydrogen or methyl such as:
   2-(3,5-bis-trifluoromethyl-phenyl)-N-[6-(2-hydroxy-ethylamino)-4-o-tolyl-pyridin-3-yl]-N-methyl-isobutyramide,
   2-(3,5-bis-trifluoromethyl-phenyl)-N-[6-(2,3-dihydro-[1,4]oxazin-4-yl)-4-o-tolyl-pyridin-3-yl]-N-methyl-isobutyramide,
   N-(6-acetylamino-4-o-tolyl-pyridin-3-yl)-2-(3,5-bis-trifluoromethyl-phenyl)-N-methyl-isobutyramide,
   N- [6-(acetyl-methyl-amino)-4-o-tolyl-pyridin-3-yl]-2-(3,5-bis-trifluoromethylphenyl)-N-methyl-isobutyramide,
   cyclopropanecarboxylic acid (5-{[2-(3,5-bis-trifluoromethyl-phenyl)-2-methylpropionyl]-methyl-amino}-4-o-tolyl-pyridin-2-yl)-amide,
   cyclopropanecarboxylic acid (5-{[2-(3,5-bis-trifluoromethyl-phenyl)-2-methylpropionyl]-methyl-amino}-4-o-tolyl-pyridin-2-yl)-methyl-amide,
   2-(3,5-bis-trifluoromethyl-phenyl)-N-(6-imidazol-1-yl-4-o-tolyl-pyridin-3-yl)-N-methyl-isobutyramide; or
   2-(3,5-bis-trifluoromethyl-phenyl)-N-[4-(2-chloro-phenyl)-6-(2-hydroxy-ethylamino)-pyridin-3-yl]-N-methyl-isobutyramide.

The most preferred compound for the use in accordance with the present invention is N-(3,5-bis-trifluoromethyl-benzyl)-N-methyl-6-(4-methyl-piperazin-1-yl)-4-o-tolyl-nicotinamide disclosed in EP-A-1,035,115.

Also preferred are compounds according to formula (I), wherein R⁴ is -(C≡C)ₘR⁷ or -(CR'=CR")ₘR⁷. Typical compounds in this group can be characterized as follows:

Compounds of formula (I), in which X is -C(O)N(CH₃)- and -(R²)ₙ is 3,5-di-CF₃ represent a first group of compounds. Exemplary preferred compounds of this group are those, wherein R³/R³ are both hydrogen and R is methyl, for example the following compounds:
N-(3,5-bis-trifluoromethyl-benzyl)-6-(4-hydroxyacetyl-piperazin-1-yl)-N-methyl-4-o-tolyl-nicotinamide,
N-(3,5-bis-trifluoromethyl-benzyl)-6-chloro-N-methyl-4-o-tolyl-nicotinamide,
N-(3,5-bis-trifluoromethyl-benzyl)-6-cyanomethyl-N-methyl-4-o-tolyl-nicotinamide,
N-(3,5-bis-trifluoromethyl-benzyl)-6-iodo-N-methyl-4-o-tolyl-nicotinamide,
4-o-tolyl-[2,4']bipyridinyl-5-carboxylic acid (3,5-bis-trifluoromethyl-benzyl)-methyl-amide,
5-[(3,5-bis-trifluoromethyl-benzyl)-methyl-carbamoyl]-4-o-tolyl-pyridine-2-carboxylic acid methyl ester,
N-(3,5-bis-trifluoromethyl-benzyl)-6-hydroxymethyl-N-methyl-4-o-tolyl-nicotinamide,
6-(5-acetyl-thiophen-2-yl)-N-(3,5-bis-trifluoromethyl-benzyl)-N-methyl-4-o-tolyl-nicotinamide,
4-o-tolyl-1',2',3',6'-tetrahydro-[2,4']bipyridinyl-5-carboxylic acid (3,5-bis-trifluoromethyl-benzyl)-methyl-amide,
N-(3,5-bis-trifluoromethyl-benzyl)-6-(4-hydroxymethyl-phenyl)-N-methyl-4-o-tolyl-nicotinamide,
2'-methyl-4-o-tolyl-[2,4']bipyridinyl-5-carboxylic acid (3,5-bis-trifluoromethylbenzyl)-methyl-amide,
N-(3,5-bis-trifluoromethyl-benzyl)-N-methyl-6-(3-methyl-[1,2,4]oxadiazol-5-yl)-4-o-tolyl-nicotinamide,
6-(3-amino-prop-1-ynyl)-N-(3,5-bis-trifluoromethyl-benzyl)-N-methyl-4-o-tolyl-nicotinamide,
(RS)-N-(3,5-bis-trifluoromethyl-benzyl)-6-(2-hydroxy-ethanesulfinylmethyl)-N-methyl-4-o-tolyl-nicotinamide,
N-(3,5-bis-trifluoromethyl-benzyl)-N-methyl-6-(1-methyl-1H-imidazol-2-yl-sulfanylmethyl)-4-o-tolyl-nicotinamide,
(RS)-N-(3,5-bis-trifluoromethyl-benzyl)-N-methyl-6-(pyridine-2-sulfinyl)-4-o-tolyl-nicotinamide,
N-(3,5-bis-trifluoromethyl-benzyl)-N-methyl-6-(pyridine-2-sulfonyl)-4-o-tolyl-nicotinamide or
N-(3,5-bis-trifluoromethyl-benzyl)-6-(3-hydroxy-propoxy)-N-methyl-4-o-tolyl-nicotinamide.

Further preferred are compounds of formula (I) wherein R⁴ is -(C≡C)ₘR⁷ or -(CR'=CR")ₘR⁷ and in which X is -N(CH₃)C(O)- and -(R²)ₙ is 3,5-di-CF₃. Exemparly preferred compounds of this group are those, wherein R³/R³ are both methyl and R is methyl, for example the following compounds:
2-(3,5-bis-trifluoromethyl-phenyl)-N-{6-[hydroxy-(2-hydroxy-ethyl)-amino]-4-o-tolyl-pyridin-3-yl}-N-methyl-isobutyramide,
2-(3,5-bis-trifluoromethyl-phenyl)-N-methyl-N- [6-(3-oxo-morpholin-4-yl)-4-o-tolyl-pyridin-3-yl] -isobutyramide,
acetic acid (5-{[2-(3,5-bis-trifluoromethyl-phenyl)-2-methyl-propionyl]-methyl-amino}-4-o-tolyl-pyridin-2-ylcarbamoyl)-methyl ester,
2-(3,5-bis-trifluoromethyl-phenyl)-N-[6-(2-hydroxy-acetylamino)-4-o-tolyl-pyridin-3-yl]-N-methyl-isobutyramide,
2-(3,5-bis-trifluoromethyl-phenyl)-N-[6-(hydroxyacetyl-methyl-amino)-4-o-tolyl-pyridin-3-yl]-N-methyl-isobutyramide,
2-(3,5-bis-trifluoromethyl-phenyl)-N-[6-(2,5-dioxo-pyrrolidin-1-yl)-4-o-tolyl-pyridin-3-yl]-N-methyl-isobutyramide,
cyclopropanecarboxylic acid (5-{ [2-(3,5-bis-trifluoromethyl-phenyl)-2-methylpropionyl]-methyl-amino}-4-o-tolyl-pyridin-2-yl)-cyclopropanecarbonyl-amide,
2-(3,5-bis-trifluoromethyl-phenyl)-N-(6-chloro-4-o-tolyl-pyridin-3-yl)-N-methyl-isobutyramide,
2-(3,5-bis-trifluoromethyl-phenyl)-N-[4-(2-chloro-phenyl)-2'-methyl-[2,4']bipyridinyl-5-yl]-N-methyl-isobutyramide,
2-(3,5-bis-trifluoromethyl-phenyl)-N-(6-ethynyl-4-o-tolyl-pyridin-3-yl)-N-methyl-isobutyramide,
2-(3,5-bis-trifluoromethyl-phenyl)-N-[6-(3-hydroxymethyl-isoxazol-5-yl)-4-o-tolyl-pyridin-3-yl]-N-methyl-isobutyramide,
2-(3,5-bis-trifluoromethyl-phenyl)-N-[6-(3-hydroxy-prop-1-ynyl)-4-o-tolyl-pyridin-3-yl] -N-methyl-isobutyramide or
(RS)-2-(3,5-bis-trifluoromethyl-phenyl)-N-[6-(3-methoxy-benzenesulfinyl)-4-o-tolyl-pyridin-3-yl]-N-methyl-isobutyramide.

Further preferred compounds of formula (I) wherein R⁴ is -(C≡C)ₘR⁷ or -(CR'=CR")ₘR⁷ are those, wherein R³/R³ are both methyl and R is chloro, for example the following compounds:
2-(3,5-bis-trifluoromethyl-phenyl)-N-{4-(2-chloro-phenyl)-6-[hydroxy-(2-hydroxy-ethyl)-amino]-pyridin-3-yl}-N-methyl-isobutyramide, or
2-(3,5-bis-trifluoromethyl-phenyl)-N-[4-(2-chloro-phenyl)-6-(3-oxo-morpholin-4-yl)-pyridin-3-yl]-N-methyl-isobutyramide.

The methods for the preparation of the compounds of formula (I) wherein R⁴ is -(C≡C)ₘR⁷ or -(CR'=CR")ₘR⁷ are described in detail in International Patent Application No. PCT/EP01/08686 filed July 27, 2001 based on European Patent Application No. 00117003.4 filed August 8, 2000.

As indicated above the NK-1 receptor antagonist in accordance with the use of the present invention may be present in the form of a prodrug.

Preferred prodrugs of the compounds of general formula (I) are N-oxides such as the following exemplary compounds:
4-{5-[(3,5-bis-trifluoromethyl-benzyl)-methyl-carbamoyl]-4-o-tolyl-pyridin-2-yl}-4-oxy-piperazine-1-carboxylic acid tert-butyl ester,
5'-[(3,5-bis-trifluoromethyl-benzyl)-methyl-carbamoyl]-4'-o-tolyl-1-oxy-3,4,5,6-tetrahydro-2H-[1,2']bipyridinyl-4-carboxylic acid ethyl ester,
(RS)-6-[3-(acetyl-methyl-amino)-1-oxo-pyrrolidin-1-yl]-N-(3,5-bis-trifluoromethyl-benzyl)-N-methyl-4-o-tolyl-nicotinamide,
N-(3,5-bis-trifluoromethyl-benzyl)-N-methyl-6-(4-oxy-morpholin-4-yl)-4-o-tolyl-nicotinamide,
N-(3,5-bis-trifluoromethyl-benzyl)-6-(1,1-dioxo-1λ⁶-4-oxy-thiomorpholin-4-yl)-N-methyl-4-o-tolyl-nicotinamide,
N-(3,5-bis-trifluoromethyl-benzyl)-6-(4-formyl-1-oxy-piperazin-1-yl)-N-methyl-4-o-tolyl-nicotinamide,
N-methyl-N-(2-methyl-naphthalen-1-yl-methyl)-6-(4-oxy-morpholin-4-yl)-4-o-tolyl-nicotinamide,
N-methyl-6-(4-oxy-morpholin-4-yl)-N-naphthalen-1-yl-methyl-4-o-tolyl-nicotinamide,
N-(2-methoxy-naphthalen-1-yl-methyl)-N-methyl-6-(4-oxy-morpholin-4-yl)-4-o-tolyl-nicotinamide,
N-(2-methoxy-benzyl)-N-methyl-6-(4-oxy-morpholin-4-yl)-4-o-tolyl-nicotinamide,
N-(5-chloro-2-methoxy-benzyl)-N-methyl-6-(4-oxy-morpholin-4-yl)-4-o-tolyl-nicotinamide,
N-(2-chloro-5-methoxy-benzyl)-N-methyl-6-morpholin-4-yl-4-o-tolyl-nicotinamide,
N-methyl-6-(4-oxy-morpholin-4-yl)-N-pentafluorophenylmethyl-4-o-tolyl-nicotinamide,
N-methyl-6-(4-oxy-morpholin-4-yl)-N-naphthalen-2-yl-methyl-4-o-tolyl-nicotinamide,
N-[2-methoxy-5-(5-trifluoromethyl-tetrazol-1-yl)-benzyl]-N-methyl-6-(4-oxy-morpholin-4-yl)-4-o-tolyl-nicotinamide,
N-(1,4-dimethoxy-naphthalen-2-yl-methyl)-N-methyl-6-(4-oxy-morpholin-4-yl)-4-o-tolyl-nicotinamide,
5'-[(3,5-bis-trifluoromethyl-benzyl)-methyl-carbamoyl]-4'-o-tolyl-1-oxy-3,4,5,6-tetrahydro-2H-[1,2']bipyridinyl-4-carboxylic acid,
2-(3,5-bis-trifluoromethyl-phenyl)-N-methyl-N-[6-(4-oxy-morpholin-4-yl)-4-o-tolyl-pyridin-3-yl]-isobutyramide,
2-(3,5-bis-trifluoromethyl-phenyl)-N-[4-(2-chloro-phenyl)-6-(4-oxy-morpholin-4-yl)-pyridin-3-yl]-N-methyl-isobutyramide,
2-(3,5-bis-trifluoromethyl-phenyl)-N-[6-(4-oxy-morpholin-4-yl)-4-o-tolyl-pyridin-3-yl]-isobutyramide,
2-(3,5-bis-trifluoromethyl-phenyl)-N-[4'-(2-chloro-phenyl)-1-oxy-3,4,5,6-tetrahydro-2H-[1,2']bipyridinyl-5'-yl]-N-methyl-isobutyramide,
2-(3,5-bis-trifluoromethyl-phenyl)-N-(6-oxy-dimethylamino-4-o-tolyl-pyridin-3-yl)-N-methyl-isobutyramide,
2-(3,5-bis-trifluoromethyl-phenyl)-N-[4-(2-chloro-phenyl)-6-oxy-dimethylamino-pyridin-3-yl]-isobutyramide,
2-(3,5-bis-trifluoromethyl-phenyl)-N-1-(4-hydroxy-1-oxy-4'-o-tolyl-3,4,5,6-tetrahydro-2H-[1,2']bipyridinyl-5'-yl)-N-methyl-isobutyramide,
2-(3,5-bis-trifluoromethyl-phenyl)-N-{6-[(2-hydroxy-ethyl)-1-oxy-methyl-amino]-4-o-tolyl-pyridin-3-yl}-N-methyl-isobutyramide,
(R)-2-(3,5-bis-trifluoromethyl-phenyl)-N-[6-(3-hydroxy-1-oxy-pyrrolidin-1-yl)-4-o-tolyl-pyridin-3-yl]-N-methyl-isobutyramide,
2-(3,5-bis-trifluoromethyl-phenyl)-N-methyl-N-[6-(4-oxy-morpholin-4-yl)-4-o-tolyl-pyridin-3-yl]-acetamide,
2-(3,5-dimethoxy-phenyl)-N-methyl-N-[6-(4-oxy-morpholin-4-yl)-4-o-tolyl-pyridin-3-yl]-acetamide; or
2-(3-fluoro-5-trifluoromethyl-phenyl)-N-methyl-N-[6-(4-oxy-morpholin-4-yl)-4-o-tolyl-pyridin-3-yl]-acetamide.

Methods for the preparation of the above-mentioned N-oxide prodrugs are described in International Patent Application No. PCT/EP01/07850 filed July 9, 2001 based on European Patent Application No. 00115287.5 filed July 14, 2000. The most preferred N-oxide prodrug of general formula (I) for the claimed use is 2-(3,5-bis-trifluoromethylphenyl)-N-methyl-N-[6-(4-oxy-morpholin-4-yl)-4-o-tolyl-pyridin-3-yl]-isobutyramide.

NK-1 receptor antagonist for use in connection with the claimed invention may be administered either alone or in combination with other therapeutic agents and are preferably formulated to a pharmaceutical composition comprising pharmaceutically acceptable carriers or diluents. The pharmaceutical preparations to be used in accordance with this invention can in addition also contain preservatives, solubilizers, stabilizers, wetting agents, emulsifiers, sweeteners, colorants, flavorants, salts for varying the osmotic pressure, buffers, masking agents or antioxidants.

NK-1 receptor antagonists can be formulated in the form of a Self-Emulsifying Drug Delivery Systems (SEDDS), which consist of mixtures of oils and surfactants, ideally isotropic, which sometimes include co-solvents. Such mixtures emulsify under conditions of gentle agitation, similar to those which would be encountered in the gastro intestinal tract. When such a formulation is released into the lumen of the gut, it disperses to form a fine emulsion, so that the drug contained in the emulsion remains in solution in the gut, avoiding the dissolution step which frequently limits the rate of absorption of hydrophobic drugs from the crystalline state. SEDDS lead to improved bioavailability and/or a more consistent temporal profile of absorption from the gut. SEDDS have been described by Pouton C.W., in Advanced Drug Delivery Reviews, 25, (1997), 47-58.

The NK-1 receptor antagonist or the pharmaceutical composition comprising it is preferably administered intravenously.

An injection solution may have the following composition:

| | |
|---|---|
| Compound of formula (I) | 1 mg |
| 1 n HCl | 20 µl |
| acetic acid | 0.5 mg |
| NaCI | 8 mg |
| phenol | 10 mg |
| 1 n NaOH | q.s. ad pH 5 |
| H₂O | q.s. ad 1 ml |

The NK-1 receptor antagonist or the pharmaceutical composition comprising it can also be administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions. The NK-1 receptor antagonist or the pharmaceutically composition comprising it can also be administered via any other suitable way known to the person skilled in the art.

The dosage can vary within wide limits and can, of course, be fitted to the individual requirements in each particular case. The dosage range for a beneficial effect in mammals depends of course on the activity of the NK-1 receptor antagonist that is used, but is usually in the range of 5 to 1000 mg/kg/d and is preferably between 25 and 100 mg/kg/d.

The pharmaceutical preparations in accordance with this invention can in addition also contain pharmaceutically inert, inorganic or organic excipients suitable for the production of tablets, coated tablets, dragees and hard gelatine capsules. Lactose, corn starch or derivatives thereof, talc, stearic acid or its salts etc. can be used as such excipients e.g. for tablets, dragées and hard gelatine capsules.

Suitable excipients for soft gelatine capsules are e.g. vegetable oils, waxes, fats, semisolid and liquid polyols etc.

Suitable excipients for the manufacture of solutions and syrups are e.g. water, polyols, saccharose, invert sugar, glucose etc.

Suitable excipients for injection solutions are e.g. water, alcohols, polyols, glycerol, vegetable oils etc.

Suitable excipients for suppositories are e.g. natural or hardened oils, waxes, fats, semi-liquid or liquid polyols etc.

Moreover, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

As indicated in the following example below the inventors have shown that NK-1 receptor antagonists, in particular N-(3,5-bis-trifluoromethyl-benzyl)-N-methyl-6-(4-methyl-piperazin-1-yl)-4-o-tolyl-nicotinamide, have the potential to reduce the development of motor and cognitive deficits followed after traumatic nerve injury and can therefore be used in the treatment and/or prevention of brain, spinal or nerve injury. While the following example illustrates the invention it is not meant to limit the scope of the claimed invention in any respect.

### EXAMPLE

The NK-1 receptor antagonist N-(3,5-bis-trifluoromethyl-benzyl)-N-methyl-6-(4-methyl-piperazin-1-yl)-4-o-tolyl-nicotinamide is blood brain barrier permeable and its effects are thought to be mediated by both peripheral and central NK-1 receptors.

Male Sprague Dawley rats (400 ± 25 g) were pretrained on both the Rotarod and Barnes maze so as to assess the effects of the compound on posttraumatic motor and cognitive outcome, respectively. After one week of training, rats were severely injured using the impact acceleration model of traumatic brain injury (2 meters) and administered either of the NK-1 receptor antagonist N-(3,5-bis-trifluoromethyl-benzyl)-N-methyl-6-(4-methyl-piperazin-1-yl)-4-o-tolyl-nicotinamide (10 mg/kg i.v., based on active, free base), the NMDA (N-methyl-D-aspartate) antagonist MK801 ((+)-10,11-dihydro-5-methyl-5H-dibenzo[a,d]cyclo-hepten-5,10-diyldiammonium maleate; Dizocilpine maleate; 0.3 mg/kg i.v., based on active, free base) or 0.9 % saline vehicle (n=10/group) at 30 minutes after injury.

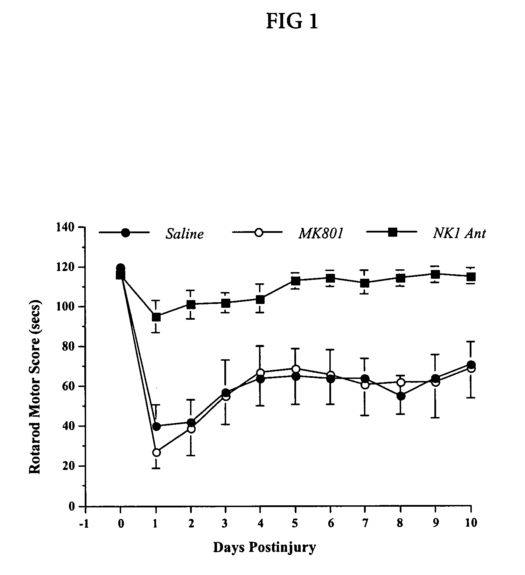

Immediately after injury, animals administered saline demonstrated a severe deficit in motor outcome, with rotarod scores declining from a preinjury score of 119 ± 1 sec to 40 ±11 sec at 24 h postinjury (Figure 1). Over the next 9 days, there was a gradual improvement in motor performance, however the deficit was always significant compared to preinjury. Rats administered the NMDA antagonist showed similar trends (Figure 1) with the motor outcome being significantly impaired at all time points post injury, and not significantly different from vehicle treated animals. In contrast, animals administered the NK-1 receptor antagonist N-(3,5-bis-trifluoromethyl-benzyl)-N-methyl-6-(4-methyl-piperazin-1-yl)-4-o-tolyl-nicotinamide demonstrated a significantly better motor outcome after brain injury than the animals treated with either saline or MK801 (Figure 1).

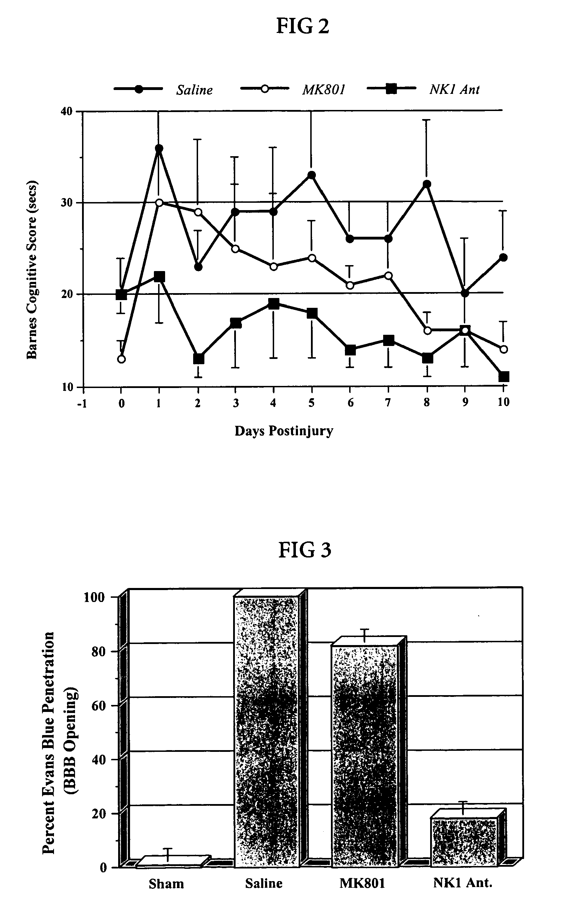

Similar results were noted with respect to cognitive performance after injury. Prior to injury, rats were trained to locate an escape tunnel (from aversive sound and light) within 20 sec. This time factor depends on the ability of the animal to learn and remember the location of the escape tunnel. After injury, this latency to escape the aversive stimuli increased to 36 sec in saline treated animals and never improved significantly over the 10 day posttraumatic assessment period (Figure 2). MK801 treated animals also demonstrated a longer latency time to escape the aversive stimuli at 24 h. However, over the ensuing 9 days, these MK801 animals did gradually improve to preinjury levels (Figure 2). In contrast, the animals administered the NK-1 receptor antagonist N-(3,5-bis-trifluoromethyl-benzyl)-N-methyl-6-(4-methyl-piperazin-1-yl)-4-o-tolyl-nicotinamide after traumatic brain injury did not demonstrate an increased latency to escape the aversive stimuli at any time point after brain injury. Indeed, their time to locate the escape tunnel after injury improved with each assessment (Figure 2), suggesting that the said NK-1 receptor antagonist was markedly protective against loss of cognitive function after traumatic brain injury.

In addition to improving posttraumatic neurologic outcome after traumatic brain injury, the NK-1 receptor antagonist N-(3,5-bis-trifluoromethyl-benzyl)-N-methyl-6-(4-methyl-piperazin-1-yl)-4-o-tolyl-nicotinamide also reduced mortality. Mortality in rats in this severe model of injury is normally between 20 and 30 %. In the saline treated animals, mortality was indeed 30 %. Administration of MK801 after trauma resulted in an increased mortality of 50 %, averaging to a 40 % mortality across these two injury groups. This high mortality was consistent with this injury level being severe as confirmed by post mortem gross histological analysis of all brains. In marked contrast to this high mortality noted in all other groups, animals treated with the NK-1 receptor antagonist N-(3,5-bis-trifluoromethyl-benzyl)-N-methyl-6-(4-methyl-piperazin-1-yl)-4-o-tolyl-nicotinamide had a 0 % mortality.

Mortality after trauma may be related to edema formation, particularly in children where profound edema has been shown to account for up to 50 % of all deaths. Edema formation in the immediate posttraumatic period is thought to be vasogenic in origin, where increased permeability of the blood brain barrier permits protein extravasation and water accumulation in the brain interstitium. We used Evans Blue extravasation as a marker of blood brain permeability after traumatic brain injury and treatment with the NK-1 receptor antagonist N-(3,5-bis-trifluoromethyl-benzyl)-N-methyl-6-(4-methyl-piperazin-1-yl)-4-o-tolyl-nicotinamide.

At 4.5 h after induction of brain injury, animals were administered i.v. Evans Blue which was allowed to circulate for 30 mins. At that time point, animals were sacrificed and their brains removed for analysis of Evans Blue penetration. The amount of Evans Blue accumulation in the brains of saline treated animals was standardised to 100 %. Relative to saline treated animals, MK801 treated animals demonstrated an 82 % Evans Blue accumulation, suggesting that the blood brain barrier in these animals was still significantly permeable (Figure 3). In marked contrast, animals treated with the NK-1 receptor antagonist N-(3,5-bis-trifluoromethyl-benzyl)-N-methyl-6-(4-methyl-piperazin-1-yl)-4-o-tolyl-nicotinamide had a significantly reduced Evans Blue penetration (18 %) indicating that the compound had markedly attenuated posttraumatic blood brain barrier permeability and associated edema formation (Figure 3). As anticipated, uninjured (sham) animals had no significant Evans Blue accumulation in brain tissue.

Finally, we note that the NK-1 receptor antagonist N-(3,5-bis-trifluoromethylbenzyl)-N-methyl-6-(4-methyl-piperazin-1-yl)-4-o-tolyl-nicotinamide had a number of general effects on the animals that was beneficial to their outcome. Following administration of the said NK-1 receptor antagonist, animals displayed a more stable respiratory pattern than their saline and MK801 treated counterparts. Indeed, animals treated with the said NK-1 receptor antagonist compound were able to be weaned of the ventilator far more quickly than any other treatment group after brain injury. This stability in respiration is thought to be due to both central effects on respiration and by the said NK-1 receptor antagonist reducing pulmonary edema formation in brain injured animals. This clearly indicates that the NK-1 receptor antagonist N-(3,5-bis-trifluoromethyl-benzyl)-N-methyl-6-(4-methyl-piperazin-1-yl)-4-o-tolyl-nicotinamide stabilises respiration and may reduce pulmonary oedema formation.

All animals after brain injury exhibit reduced exploratory behaviour and limited self grooming, suggesting a posttraumatic depression. In animals treated with the NK-1 receptor antagonist N-(3,5-bis-trifluoromethyl-benzyl)-N-methyl-6-(4-methyl-piperazin-1-yl)-4-o-tolyl-nicotinamide, exploratory behaviour and self-grooming were not significantly different from non-injured animals. Although the mechanisms are unknown, this action is thought to be mediated through central effects since this improvement is not observed with NK-1 receptor antagonists that have limited CNS penetration. This suggests that the NK-1 receptor antagonist N-(3,5-bis-trifluoromethyl-benzyl)-N-methyl-6-(4-methyl-piperazin-1-yl)-4-o-tolyl-nicotinamide may also be useful for attenuating posttraumatic depression following brain injury.

The results summarised above indicate that the NK-1 receptor antagonist N-(3,5-bis-trifluoromethyl-benzyl)-N-methyl-6-(4-methyl-piperazin-1-yl)-4-o-tolyl-nicotinamide can reduce mortality, motor and cognitive deficits after brain injury. Nonetheless, it has been shown previously (International Patent Application No. PCT/AU01/00046) that the neuroprotective action of NK-1 receptor antagonists can be enhanced with the addition of pharmacologic doses of magnesium to the i.v. solution. Therefore, the NK-1 receptor antagonist as used in accordance with the present invention, such as N-(3,5-bis-trifluoromethyl-benzyl)-N-methyl-6-(4-methyl-piperazin-1-yl)-4-o-tolyl-nicotinamide, is preferably co-administered with pharmacologic doses of magnesium salts (10-100 mg/kg) in order to enhance the neuroprotective properties.

## Claims

1. The use of an NK-1 receptor antagonist for the manufacture of a medicament for the treatment and/or prevention of brain, spinal or nerve injury, wherein the NK-1 receptor antagonist is a compound of the general formula (I) 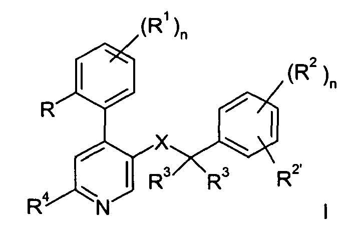 wherein
R is hydrogen, lower alkyl, lower alkoxy, halogen or trifluoromethyl;
R¹ is hydrogen or halogen; or
R and R¹ may be together -CH=CH-CH=CH-;
R² and R²' are independently from each other hydrogen, halogen, trifluoromethyl, lower alkyl, lower alkoxy or cyano; or
R² and R^{2'} may be together -CH=CH-CH=CH-, optionally substituted by one or two substituents selected from lower alkyl, halogen or lower alkoxy;
R³ is, independently from each other if occurring twice, hydrogen, lower alkyl or may, if occurring twice, form together with the carbon atom to which they are attached a cycloalkyl group;
R⁴ is (A) hydrogen, -N(R⁵)₂, -N(R⁵)(CH₂)ₙOH, -N(R⁵)S(O)₂-lower alkyl, -N(R⁵)S(O)₂₋phenyl, -N=CH-N(R⁵)₂, -N(R⁵)C(O)R⁵, a cyclic tertiary amine of the group 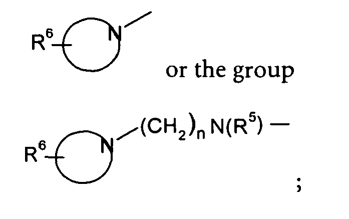
or (B) R⁴ is -(C≡C)ₘR⁷ or -(CR'=CR")ₘR⁷
wherein R⁷ is
a) halogen,
b) cyano, or the following groups:
c) -(CR'R")ₘ-R⁸,
d) -C(O)NR'R",
e) -C(O)O(CH₂)ₙR⁸,
f) -C(O)R⁸,
g) -N(OH)-(CH₂)ₙR⁸,
h) -NR'C(O)-(CH₂)ₙR⁸,
i) -N[C(O)-R']₂,
j) -OR⁹,
k) -(CH₂)ₙ-SR⁹, -(CH₂)ₙ-S(O)R⁹, or -(CH₂)ₙ-S(O)₂R⁹,
l) aryl, optionally substituted by one or more substituents, selected from halogen, trifluoromethyl, lower alkyl, lower alkoxy, cyano, hydroxy, -NR'R", nitro, -(CH₂)ₘOR', -C(O)NR'R", -C(O)OR' or -C(O)R',
m) is a five or six membered heteroaryl group, containing one to four heteroatoms, selected from N, O or S and may be optionally substituted by one or more substituents, selected from halogen, trifluoromethyl, lower alkyl, lower alkoxy, cyano, hydroxy, -NR'R", nitro, -(CH₂)ₘOR', -C(O)OR', -C(O)NR'R" or -C(O)R',
n) is a five or six membered saturated cyclic tertiary amine of the group 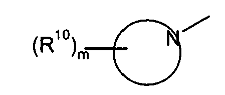 which may contain one additional heteroatom, selected from N, O or S,
R'/R" are independently from each other hydrogen, hydroxy, lower alkyl, cycloalkyl or aryl, wherein the lower alkyl, cycloalkyl or aryl group may be optionally substituted by one or more substituents, selected from halogen, trifluoromethyl, lower alkyl, lower alkoxy, cyano, hydroxy, -NR"'R"", nitro, -(CH₂)ₘOR'", -C(O)NR'"R"", -C(O)OR'" or -C(O)R'",
R'"/R"" are independently from each other hydrogen, lower alkyl, cycloalkyl or aryl,
R⁸ is hydrogen, cyano, hydroxy, halogen, trifluoromethyl, -C(O)OR', -OC(O)R' or aryl, optionally substituted by one or more substituents, selected from halogen, trifluoromethyl, lower alkyl, lower alkoxy, cyano, hydroxy, -NR'R", nitro, -(CH₂)ₘOR', -C(O)NR'R", -C(O)OR' or -C(O)R', or is a five or six membered heteroaryl group, containing one to four heteroatoms, selected from N, O or S and may be optionally substituted by one or more substituents, selected from halogen, trifluoromethyl, lower alkyl, lower alkoxy, cyano, hydroxy, -NR'R", nitro, -(CH₂)ₘOR', -C(O)NR'R", -C(O)OR' or -C(O)R',
R⁹ is hydrogen, lower alkyl, trifluoromethyl, or aryl, wherein the lower alkyl or aryl group may be optionally substituted by one or more substituents, selected from halogen, trifluoromethyl, lower alkyl, lower alkoxy, cyano, hydroxy, -NR'R", nitro, -C(O)NR'R", -(CH₂)ₘOR', -C(O)OR' or -C(O)R', or is a five or six membered heteroaryl group, containing one to four heteroatoms, selected from N, O or S and may be optionally substituted by one or more substituents, selected from halogen, trifluoromethyl, lower alkyl, lower alkoxy, cyano, hydroxy, -NR'R", nitro, -(CH₂)ₘOR', -C(O)NR'R", -C(O)OR' or -C(O)R',
R¹⁰ is -C(O)-(CH₂)ₘOH or an oxo group;
or (C) R⁴ is an N-oxide of the general formula 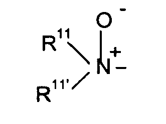 wherein R¹¹ and R^{11'} are independently from each other -(CH₂)ₚOR¹² or lower alkyl, wherein R¹² is hydrogen, lower alkyl or phenyl;
or
R¹¹ and R^{11'} form together with the N-atom to which they are attached a cyclic tertiary amine of the group 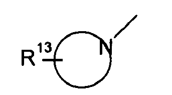 wherein R¹³ is hydrogen, hydroxy, lower alkyl, lower alkoxy, -(CH₂)ₚOH, -COOR³, -CON(R³)₂ , -N(R³)CO-lower alkyl or -C(O)R³;
R⁵ is, independently from each other, hydrogen, C₃-₆-cycloalkyl, benzyl, phenyl or lower alkyl;
R⁶ is hydrogen, hydroxy, lower alkyl, -(CH₂)ₙCOO-lower alkyl, -N(R⁵)CO-lower alkyl, hydroxy-lower alkyl, cyano, -(CH₂)ₙO(CH₂)ₙOH, -CHO or a 5-or 6 membered heterocyclic group, optionally bonded via an alkylene group;
X is -C(O)N(R⁵)-, -(CH₂)ₘO-, -O(CH₂)ₘ-, -(CH₂)ₘN(R⁵)-, -N(R⁵)C(O)-, or -N(R⁵)(CH₂)ₘ-;
n is 0, 1, 2, 3 or 4;
m is 1 or 2; and
p is 1, 2 or 3;
and the pharmaceutically acceptable acid addition salts and the prodrugs thereof.

2. The use according to claim 1, wherein the NK-1 receptor antagonist is a compound of general formula (I) in which R⁴ has the meaning (A) as defined in claim 1.

3. The use according to claim 1, wherein the NK-1 receptor antagonist is a compound of general formula (I) in which R⁴ has the meaning (B) as defined in claim 1.

4. The use according to claim 1, wherein the NK-1 receptor antagonist is a compound of general formula (I) in which R⁴ has the meaning (C) as defined in claim 1.

5. The use according to claim 1 or 2, wherein the NK-1 receptor antagonist is a compound of general formula (I) in which X is -C(O)N(R⁵)- and wherein R⁵ is methyl, ethyl or cyclopropyl.

6. The use according to claim 5, wherein the compound is selected from the group consisting of
N-(3,5-bis-trifluoromethyl-benzyl)-N-methyl-4-o-tolyl-nicotinamide,
N-(3,5-bis-trifluoromethyl-benzyl)-N-methyl-4-(2-chloro-phenyl)-nicotinamide,
N-(3,5-bis-trifluoromethyl-benzyl)-N-methyl-4-(2-trifluoromethyl-phenyl)-nicotinamide,
N-(3,5-bis-trifluoromethyl-benzyl)-N-methyl-4-(2-fluoro-phenyl)-nicotinamide,
N-(3,5-bis-trifluoromethyl-benzyl)-N-methyl-4-(2-methoxy-phenyl)-nicotinamide,
N-(3,5-bis-trifluoromethyl-benzyl)-N-methyl-4-phenyl-nicotinamide,
N-(3,5-bis-trifluoromethyl-benzyl)-N-ethyl-4-o-tolyl-nicotinamide,
N-(3,5-bis-trifluoromethyl-benzyl)-N-cyclopropyl-4-o-tolyl-nicotinamide,
N-[1-(3,5-bis-trifluoromethyl-phenyl)-ethyl]-N-methyl-4-o-tolyl-nicotinamide,
N-(3,5-di-fluorobenzyl)-N-methyl-4-o-tolyl-nicotinamide,
N-(3,5-di-chlorobenzyl)-N-methyl-4-o-tolyl-nicotinamide,
N-(3,5-bis-trifluoromethyl-benzyl)-N-methyl-6-(4-methyl-piperazin-1-yl)-4-o-tolyl-nicotinamide,
2'-methyl-5-(4-methyl-piperazin-1-yl)-biphenyl-2-carboxylic acid-(3,5-bis-trifluoromethyl-benzyl)-methyl-amide,
N-(3,5-bis-trifluoromethyl-benzyl)-N-methyl-6-(4-methyl-piperazin-1-yl)-4-naphthalen-1-yl-nicotinamide,
(4-{5-[(3,5-bis-trifluoromethyl-benzyl)-methyl-carbamoyl]-4-o-tolyl-pyridin-2-yl}-piperazin-1-yl)-acetic acid ethyl ester,
5'-[(3,5-bis-trifluoromethyl-benzyl)-methyl-carbamoyl]-4'-o-tolyl-3,4,5,6-tetrahydro-2H-[1,2']bipyridinyl-4-carboxylic acid ethyl ester,
N-(3,5-bis-trifluoromethyl-benzyl)-N-methyl-6-(4-propyl-piperazin-1-yl)-4-o-tolyl-nicotinamide,
(RS)-6-[3-(acetyl-methyl-amino)-pyrrolidin-1-yl]-N-(3,5-bis-trifluoromethylbenzyl)-N-methyl-4-o-tolyl-nicotinamide,
N-(3,5-bis-trifluoromethyl-benzyl)-N-methyl-6-[methyl-(2-morpholin-4-yl-ethyl)-amino]-4-o-tolyl-nicotinamide,
N-(3,5-bis-trifluoromethyl-benzyl)-N-methyl-6-morpholin-4-yl-4-o-tolyl-nicotinamide,
N-(3,5-bis-trifluoromethyl-benzyl)-N-methyl-6-thiomorpholin-4-yl-4-o-tolyl-nicotinamide,
N-(3,5-bis-trifluoromethyl-benzyl)-N-methyl-6-(1-oxo-1λ⁴-thiomorpholin-4-yl)-4-o-tolyl-nicotinamide,
N-(3,5-bis-trifluoromethyl-benzyl)-6-1,1-dioxo-1λ⁶-thiomorpholin-4-yl)-N-methyl-4-o-tolyl-nicotinamide,
N-(3,5-bis-trifluoromethyl-benzyl)-N-methyl-6-piperazin-1-yl-4-o-tolyl-nicotinamide,
N-(3,5-bis-trifluoromethyl-benzyl)-6-[4-(2-hydroxy-ethyl)-piperazin-1-yl]-N-methyl-4-o-tolyl-nicotinamide,
N-(3,5-bis-trifluoromethyl-benzyl)-6-(4-cyanomethyl-piperazin-1-yl)-N-methyl-4-o-tolyl-nicotinamide,
N-(3,5-bis-trifluoromethyl-benzyl)-6-{4-[2-(2-hydroxy-ethoxy)-ethyl]-piperazin-1-yl}-N-methyl-4-o-tolyl-nicotinamide,
N-(3,5-bis-trifluoromethyl-benzyl)-N-methyl-6-(4-[1,2,4]oxadiazol-3-yl-methyl-piperazin-1-yl)-4-o-tolyl-nicotinamide,
N-(3,5-bis-trifluoromethyl-benzyl)-N-methyl-6-[4-(5-oxo-4,5-dihydro-1H-[1,2,4]triazol-3-yl-methyl)-piperazin-1-yl]-4-o-tolyl-nicotinamide,
N-(3,5-bis-trifluoromethyl-benzyl)-6-(4-formyl-piperazin-1-yl)-N-methyl-4-o-tolyl-nicotinamide, and
N-methyl-N-(2-methyl-naphthalen-1-yl-methyl)-6-morpholin-4-yl-4-o-tolyl-nicotinamide;
or a pharmaceutically acceptable acid addition salt thereof.

7. The use according to claim 1 or 2, wherein the NK-1 receptor antagonist is a compound of general formula (I), wherein X is -N(R⁵)-C(O)- and R⁵ is hydrogen or methyl.

8. The use according to claim 7, wherein the compound is selected from the group consisting of
2-(3,5-bis-trifluoromethyl-phenyl)-N-methyl-N-[6-(4-methyl-piperazin-1-yl)-4-o-tolyl-pyridin-3-yl]-isobutyramide,
2-(3,5-bis-trifluoromethyl-phenyl)-N-[4-(2-chloro-phenyl)-6-(4-methyl-piperazin-1-yl)-pyridin-3-yl]-N-methyl-isobutyramide,
2-(3,5-bis-trifluoromethyl-phenyl)-N-[4-(4-fluoro-2-methyl-phenyl)-6-(4-methyl-piperazin-1-yl)-pyridin-3-yl]-N-methyl-isobutyramide,
2-(3,5-bis-trifluoromethyl-phenyl)-N-[4-(2-chloro-phenyl)-pyridin-3-yl]-N-methyl-isobutyramide,
2-(3,5-bis-trifluoromethyl-phenyl-N-methyl-N-(4-o-tolyl-pyridin-3-yl)-isobutyramide,
2-(3,5-bis-trifluoromethyl-phenyl)-N-(4-o-tolyl-pyridin-3-yl)-isobutyramide,
2-(3,5-bis-trifluoromethyl-phenyl)-N-methyl-N-(4-o-tolyl-pyridin-3-yl)-acetamide,
2-(3,5-bis-trifluoromethyl-phenyl)-N-methyl-N-(4-o-tolyl-pyridin-3-yl)-propionamide,
2-(3,5-bis-trifluoromethyl-phenyl)-N-methyl-N-(6-morpholin-4-yl-4-o-tolyl-pyridin-3-yl)-isobutyramide,
2-(3,5-bis-trifluoromethyl-phenyl)-N- [4-(2-chloro-phenyl)-6-morpholin-4-yl-pyridin-3-yl] -N-methyl-isobutyramide,
2-(3,5-bis-trinuoromethyl-phenyl)-N-methyl-N-{6-[methyl-(2-morpholin-4-yl-ethyl)-amino]-4-o-tolyl-pyridin-3-yl}-isobutyramide,
2-(3,5-bis-trifluoromethyl-phenyl)-N-methyl-N-[6-(4-pyrimidin-2-yl-piperazin-1-yl)-4-o-tolyl-pyridin-3-yl] -isobutyramide,
2-(3,5-bis-trifluoromethyl-phenyl)-N-(6-morpholin-4-yl-4-o-tolyl-pyridin-3-yl)-isobutyramide,
2-(3,5-bis-trifluoromethyl-phenyl)-N-[4-(2-chloro-phenyl)-6-dimethylamino-pyridin-3-yl] -isobutyramide,
2-(3,5-bis-trifluoromethyl-phenyl)-N-methyl-N-(6-piperazin-1-yl-4-o-tolyl-pyridin-3-yl)-isobutyramide,
2-(3,5-bis-trifluoromethyl-phenyl)-N-(4-hydroxy-4'-o-tolyl-3,4,5,6-tetrahydro-2H-[1,2']bipyridinyl-5'-yl)-N-methyl-isobutyramide,
2-(3,5-bis-trifluoromethyl-phenyl)-N-{6-[(2-hydroxy-ethyl)-methyl-amino]-4-o-tolyl-pyridin-3-yl}-N-methyl-isobutyramide,
(R)-2-(3,5-bis-trifluoromethyl-phenyl)-N-[6-(3-hydroxy-pyrrolidin-1-yl)-4-o-tolyl-pyridin-3-yl]-N-methyl-isobutyramide,
2-(3,5-bis-trifluoromethyl-phenyl)-N-methyl-N-(6-morpholin-4-yl-4-o-tolyl-pyridin-3-yl)-acetamide, and
[2-(3,5-bis-trifluoromethyl-phenyl)-2-methyl-propyl]-[4-(4-fluoro-2-methylphenyl)-6-(4-methyl-piperazin-1-yl)-pyridin-3-yl]-methylamine;
or a pharmaceutically acceptable acid addition salt thereof.

9. The use according to claim 5, wherein the compound is selected from the group consisting of
N-(3,5-bis-trifluoromethyl-benzyl)-N-methyl-4-o-tolyl-6-[1,2,4]triazol-1-yl-nicotinamide,
N-(3,5-bis-trifluoromethyl-benzyl)-6-(2-hydroxy-ethylamino)-N-methyl-4-o-tolyl-nicotinamide,
4-hydroxy-4'-o-tolyl-3,4,5,6-tetrahydro-2H-[1,2']bipyridinyl-5'-carboxylic acid (3,5-bis-trifluoromethyl-benzyl)-methyl-amide,
4-(2-hydroxy-ethoxy)-4'-o-tolyl-3,4,5,6-tetrahydro-2H-[1,2']bipyridinyl-5'-carboxylic acid (3,5-bis-trifluoromethyl-benzyl)-methyl-amide,
(R)-N-(3,5-bis-trifluoromethyl-benzyl)-6-(3-hydroxy-pyrrolidin-1-yl)-N-methyl-4-o-tolyl-nicotinamide, and
4'-(2-chloro-phenyl)-4-hydroxy-3,4,5,6-tetrahydro-2H-[1,2']bipyridinyl-5'-carboxylic acid (3,5-bis-trifluoromethyl-benzyl)-methyl-amide;
or a pharmaceutically acceptable acid addition salt thereof.

10. The use according to claim 7, wherein the compound is selected from the group consisting of
2-(3,5-bis-trifluoromethyl-phenyl)-N-[6-(2-hydroxy-ethylamino)-4-o-tolyl-pyridin-3-yl]-N-methyl-isobutyramide,
2-(3,5-bis-trifluoromethyl-phenyl)-N-[6-(2,3-dihydro-[1,4] oxazin-4-yl)-4-o-tolyl-pyridin-3-yl]-N-methyl-isobutyramide,
N-(6-acetylamino-4-o-tolyl-pyridin-3-yl)-2-(3,5-bis-trifluoromethyl-phenyl)-N-methyl-isobutyramide,
N-[6-(acetyl-methyl-amino)-4-o-tolyl-pyridin-3-yl]-2-(3,5-bis-trifluoromethylphenyl)-N-methyl-isobutyramide,
cyclopropanecarboxylic acid (5-{[2-(3,5-bis-trifluoromethyl-phenyl)-2-methylpropionyl]-methyl-amino}-4-o-tolyl-pyridin-2-yl)-amide,
cyclopropanecarboxylic acid (5-{[2-(3,5-bis-trifluoromethyl-phenyl)-2-methylpropionyl]-methyl-amino}-4-o-tolyl-pyridin-2-yl)-methyl-amide,
2-(3,5-bis-trifluoromethyl-phenyl)-N-(6-imidazol-1-yl-4-o-tolyl-pyridin-3-yl)-N-methyl-isobutyramide, and
2-(3,5-bis-trifluoromethyl-phenyl)-N-[4-(2-chloro-phenyl)-6-(2-hydroxyethylamino)-pyridin-3-yl]-N-methyl-isobutyramide;
or a pharmaceutically acceptable acid addition salt thereof.

11. The use according to claim 1 or 3, wherein the NK-1 receptor antagonist is a compound of general formula (I), wherein R⁴ is -(C≡C)ₘR⁷ or -(CR'=CR")ₘR⁷.

12. The use according to claim 11, wherein the NK-1 receptor antagonist is a compound according to formula (I), wherein in R⁴ is -(C≡C)ₘR⁷ or -(CR'=CR")ₘR⁷ and X is -C(O)N(CH₃)- and (R²)ₙ is 3,5-di-CF₃.

13. The use according to claim 12, wherein the compound is selected from the group consisting of
N-(3,5-bis-trifluoromethyl-benzyl)-6-(4-hydroxyacetyl-piperazin-1-yl)-N-methyl-4-o-tolyl-nicotinamide,
N-(3,5-bis-trifluoromethyl-benzyl)-6-chloro-N-methyl-4-o-tolyl-nicotinamide,
N-(3,5-bis-trifluoromethyl-benzyl)-6-cyanomethyl-N-methyl-4-o-tolyl-nicotinamide,
N-(3,5-bis-trifluoromethyl-benzyl)-6-iodo-N-methyl-4-o-tolyl-nicotinamide,
4-o-tolyl-[2,4']bipyridinyl-5-carboxylic acid (3,5-bis-trifluoromethyl-benzyl)-methyl-amide,
5-[(3,5-bis-trifluoromethyl-benzyl)-methyl-carbamoyl]-4-o-tolyl-pyridine-2-carboxylic acid methyl ester,
N-(3,5-bis-trifluoromethyl-benzyl)-6-hydroxymethyl-N-methyl-4-o-tolyl-nicotinamide,
6-(5-acetyl-thiophen-2-yl)-N-(3,5-bis-trifluoromethyl-benzyl)-N-methyl-4-o-tolyl-nicotinamide,
4-o-tolyl-1',2',3',6'-tetrahydro-[2,4']bipyridinyl-5-carboxylic acid (3,5-bis-trifluoromethyl-benzyl)-methyl-amide,
N-(3,5-bis-trifluoromethyl-benzyl)-6-(4-hydroxymethyl-phenyl)-N-methyl-4-o-tolyl-nicotinamide,
2'-methyl-4-o-tolyl-[2,4']bipyridinyl-5-carboxylic acid (3,5-bis-trifluoromethylbenzyl)-methyl-amide,
N-(3,5-bis-trifluoromethyl-benzyl)-N-methyl-6-(3-methyl-[1,2,4] oxadiazol-5-yl)-4-o-tolyl-nicotinamide,
6-(3-amino-prop-1-ynyl)-N-(3,5-bis-trifluoromethyl-benzyl)-N-methyl-4-o-tolyl-nicotinamide,
(RS)-N-(3,5-bis-trifluoromethyl-benzyl)-6-(2-hydroxy-ethanesulfinylmethyl)-N-methyl-4-o-tolyl-nicotinamide,
N-(3,5-bis-trifluoromethyl-benzyl)-N-methyl-6-(1-methyl-1H-imidazol-2-yl-sulfanylmethyl)-4-o-tolyl-nicotinamide,
(RS)-N-(3,5-bis-trifluoromethyl-benzyl)-N-methyl-6-(pyridine-2-sulfinyl)-4-o-tolyl-nicotinamide,
N-(3,5-bis-trifluoromethyl-benzyl)-N-methyl-6-(pyridine-2-sulfonyl)-4-o-tolyl-nicotinamide, and
N-(3,5-bis-trifluoromethyl-benzyl)-6-(3-hydroxy-propoxy)-N-methyl-4-o-tolyl-nicotinamide;
or a pharmaceutically acceptable acid addition salt thereof.

14. The use according to claim 11, wherein the NK-1 receptor antagonist is a compound of general formula (I), wherein R⁴ is -(C≡C)ₘR⁷ or -(CR'=CR")ₘR⁷ and X is -N(CH₃)C(O)- and (R²)ₙ is 3,5-di-CF₃.

15. The use according to claim 14, wherein the compound is selected from the group consisting of
2-(3,5-bis-trifluoromethyl-phenyl)-N-{6-[hydroxy-(2-hydroxy-ethyl)-amino]-4-o-tolyl-pyridin-3-yl}-N-methyl-isobutyramide,
2-(3,5-bis-trifluoromethyl-phenyl)-N-methyl-N-[6-(3-oxo-morpholin-4-yl)-4-o-tolyl-pyridin-3-yl]-isobutyramide,
acetic acid (5-{[2-(3,5-bis-trifluoromethyl-phenyl)-2-methyl-propionyl]-methyl-amino}-4-o-tolyl-pyridin-2-ylcarbamoyl)-methyl ester,
2-(3,5-bis-trifluoromethyl-phenyl)-N-[6-(2-hydroxy-acetylamino)-4-o-tolyl-pyridin-3-yl]-N-methyl-isobutyramide,
2-(3,5-bis-trifluoromethyl-phenyl)-N-[6-(hydroxyacetyl-methyl-amino)-4-o-tolyl-pyridin-3-yl]-N-methyl-isobutyramide,
2-(3,5-bis-trifluoromethyl-phenyl)-N-[6-(2,5-dioxo-pyrrolidin-1-yl)-4-o-tolyl-pyridin-3-yl]-N-methyl-isobutyramide,
cyclopropanecarboxylic acid (5-{[2-(3,5-bis-trifluoromethyl-phenyl)-2-methylpropionyl]-methyl-amino}-4-o-tolyl-pyridin-2-yl)-cyclopropanecarbonyl-amide,
2-(3,5-bis-trifluoromethyl-phenyl)-N-(6-chloro-4-o-tolyl-pyridin-3-yl)-N-methyl-isobutyramide,
2-(3,5-bis-trifluoromethyl-phenyl)-N-[4-(2-chloro-phenyl)-2'-methyl-[2,4']bipyridinyl-5-yl]-N-methyl-isobutyramide,
2-(3,5-bis-trifluoromethyl-phenyl)-N-(6-ethynyl-4-o-tolyl-pyridin-3-yl)-N-methyl-isobutyramide,
2-(3,5-bis-trifluoromethyl-phenyl)-N-[6-(3-hydroxymethyl-isoxazol-5-yl)-4-o-tolyl-pyridin-3-yl]-N-methyl-isobutyramide,
2-(3,5-bis-trifluoromethyl-phenyl)-N-[6-(3-hydroxy-prop-1-ynyl)-4-o-tolyl-pyridin-3-yl]-N-methyl-isobutyramide, and
(RS)-2-(3,5-bis-trifluoromethyl-phenyl)-N-[6-(3-methoxy-benzenesulfinyl)-4-o-tolyl-pyridin-3-yl]-N-methyl-isobutyramide;
or a pharmaceutically acceptable acid addition salt thereof.

16. The use according to claim 11, wherein the NK-1 receptor antagonist is a compound of general formula (I), wherein R⁴ is -(C≡C)ₘR^{11'} or -(CR'=CR")ₘR^{11'} and R³ and R^{3'} are both methyl and R is chloro.

17. The use according to claim 16, wherein the compound is 2-(3,5-bis-trifluoromethylphenyl)-N-{4-(2-chloro-phenyl)-6-[hydroxy-(2-hydroxy-ethyl)-amino]-pyridin-3-yl}-N-methyl-isobutyramide or is 2-(3,5-bis-trifluoromethyl-phenyl)-N-[4-(2-chloro-phenyl)-6-(3-oxo-morpholin-4-yl)-pyridin-3-yl]-N-methyl-isobutyramide or is a pharmaceutically acceptable acid addition salt thereof..

18. The use according to claim 1 or 4, wherein the NK-1 receptor antagonist is a compound of general formula (I), wherein R⁴ is an N-oxide of the general formula 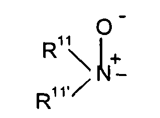 and X is -C(O)N(R⁵)- and R⁵ is methyl or X is -N(R⁵)-C(O)- and R⁵ is hydrogen or methyl and R¹¹ and R^{11'} have the meaning specified in claim 1.

19. The use according to claim 18, wherein the compound is selected from the group consisting of.
4-{5-[(3,5-bis-trifluoromethyl-benzyl)-methyl-carbamoyl]-4-o-tolyl-pyridin-2-yl}-4-oxy-piperazine-1-carboxylic acid tert-butyl ester,
5'-[(3,5-bis-trifluoromethyl-benzyl)-methyl-carbamoyl]-4'-o-tolyl-1-oxy-3,4,5,6-tetrahydro-2H-[1,2']bipyridinyl-4-carboxylic acid ethyl ester,
(RS)-6-[3-(acetyl-methyl-amino)-1-oxo-pyrrolidin-1-yl]-N-(3,5-bis-trifluoromethyl-benzyl)-N-methyl-4-o-tolyl-nicotinamide,
N-(3,5-bis-trifluoromethyl-benzyl)-N-methyl-6-(4-oxy-morpholin-4-yl)-4-o-tolyl-nicotinamide,
N-(3,5-bis-trifluoromethyl-benzyl)-6-(1,1-dioxo-1λ⁶-4-oxy-thiomorpholin-4-yl)-N-methyl-4-o-tolyl-nicotinamide,
N-(3,5-bis-trifluoromethyl-benzyl)-6-(4-formyl-1-oxy-piperazin-1-yl)-N-methyl-4-o-tolyl-nicotinamide,
N-methyl-N-(2-methyl-naphthalen-1-yl-methyl)-6-(4-oxy-morpholin-4-yl)-4-o-tolyl-nicotinamide,
N-methyl-6-(4-oxy-morpholin-4-yl)-N-naphthalen-1-yl-methyl-4-o-tolyl-nicotinamide,
N-(2-methoxy-naphthalen-1-yl-methyl)-N-methyl-6-(4-oxy-morpholin-4-yl)-4-o-tolyl-nicotinamide,
N-(2-methoxy-benzyl)-N-methyl-6-(4-oxy-morpholin-4-yl)-4-o-tolyl-nicotinamide,
N-(5-chloro-2-methoxy-benzyl)-N-methyl-6-(4-oxy-morpholin-4-yl)-4-o-tolyl-nicotinamide,
N-(2-chloro-5-methoxy-benzyl)-N-methyl-6-morpholin-4-yl-4-o-tolyl-nicotinamide,
N-methyl-6-(4-oxy-morpholin-4-yl)-N-pentafluorophenylmethyl-4-o-tolyl-nicotinamide,
N-methyl-6-(4-oxy-morpholin-4-yl)-N-naphthalen-2-yl-methyl-4-o-tolyl-nicotinamide,
N-[2-methoxy-5-(5-trifluoromethyl-tetrazol-1-yl)-benzyl]-N-methyl-6-(4-oxy-morpholin-4-yl)-4-o-tolyl-nicotinamide,
N-(1,4-dimethoxy-naphthalen-2-yl-methyl)-N-methyl-6-(4-oxy-morpholin-4-yl)-4-o-tolyl-nicotinamide,
5'-[(3,5-bis-trifluoromethyl-benzyl)-methyl-carbamoyl]-4'-o-tolyl-1-oxy-3,4,5,6-tetrahydro-2H-[1,2']bipyridinyl-4-carboxylic acid,
2-(3,5-bis-trifluoromethyl-phenyl)-N-methyl-N-[6-(4-oxy-morpholin-4-yl)-4-o-tolyl-pyridin-3-yl]-isobutyramide,
2-(3,5-bis-trifluoromethyl-phenyl)-N-[4-(2-chloro-phenyl)-6-(4-oxy-morpholin-4-yl)-pyridin-3-yl]-N-methyl-isobutyramide,
2-(3,5-bis-trifluoromethyl-phenyl)-N-[6-(4-oxy-morpholin-4-yl)-4-o-tolyl-pyridin-3-yl]-isobutyramide,
2-(3,5-bis-trifluoromethyl-phenyl)-N-[4'-(2-chloro-phenyl)-1-oxy-3,4,5,6-tetrahydro-2H-[1,2']bipyridinyl-5'-yl]-N-methyl-isobutyramide,
2-(3,5-bis-trifluoromethyl-phenyl)-N-(6-oxy-dimethylamino-4-o-tolyl-pyridin-3-yl)-N-methyl-isobutyramide,
2-(3,5-bis-trifluoromethyl-phenyl)-N-[4-(2-chloro-phenyl)-6-oxy-dimethylamino-pyridin-3-yl]-isobutyramide,
2-(3,5-bis-trifluoromethyl-phenyl)-N-1-(4-hydroxy-1-oxy-4'-o-tolyl-3,4,5,6-tetrahydro-2H-[1,2']bipyridinyl-5'-yl)-N-methyl-isobutyramide,
2-(3,5-bis-trifluoromethyl-phenyl)-N-{6-[(2-hydroxy-ethyl)-1-oxy-methyl-amino]-4-o-tolyl-pyridin-3-yl}-N-methyl-isobutyramide,
(R)-2-(3,5-bis-trifluoromethyl-phenyl)-N-[6-(3-hydroxy-1-oxy-pyrrolidin-1-yl)-4-o-tolyl-pyridin-3-yl]-N-methyl-isobutyramide,
2-(3,5-bis-trifluoromethyl-phenyl)-N-methyl-N-[6-(4-oxy-morpholin-4-yl)-4-o-tolyl-pyridin-3-yl]-acetamide,
2-(3,5-dimethoxy-phenyl)-N-methyl-N-[6-(4-oxy-morpholin-4-yl)-4-o-tolyl-pyridin-3-yl]-acetamide, and
2-(3-fluoro-5-trifluoromethyl-phenyl)-N-methyl-N-[6-(4-oxy-morpholin-4-yl)-4-o-tolyl-pyridin-3-yl]-acetamide;
or a pharmaceutically acceptable acid addition salt thereof.

20. The use according to claim 1, wherein the NK-1 receptor antagonist is 2-(3,5-bis-trifluoromethyl-phenyl)-N-methyl-N-(6-morpholin-4-yl-4-o-tolyl-pyridin-3-yl)-isobutyramide or is a pharmaceutically acceptable acid addition salt thereof.

21. The use according to claim 1, wherein the NK-1 receptor antagonist is 2-(3,5-bis-trifluoromethyl-phenyl)-N-methyl-N- [6-(4-oxy-morpholin-4-yl)-4-o-tolyl-pyridin-3-yl]-isobutyramide or is a pharmaceutically acceptable acid addition salt thereof.

22. The use according to any one of claims 1 to 21 wherein additionally a pharmacologic dose of magnesium is provided.
